# EUROPÄISCHE PATENTSCHRIFT

(11) **EP 3 836 983 B1**
(45) Veröffentlichungstag und Bekanntmachung des Hinweises auf die Patenterteilung: **07.06.2023**
(21) Anmeldenummer: 19753086.8
(22) Anmeldetag: 13.08.2019
(51) Int. Cl.: A61M 1/06

(54) **BABYFLASCHE MIT FLASCHENAUFSATZ**
BABY BOTTLE WITH BOTTLE ATTACHMENT
BIBERON POURVU DE PIÈCE RAPPORTÉE POUR BOUTEILLE

(30) Priorität: 16.08.2018 EP 18189368
(43) Veröffentlichungstag der Anmeldung: 23.06.2021
(73) Patentinhaber: Medela Holding AG, 6340 Baar (CH)
(72) Erfinder: THÜRING, Martin, 5643 Sins (CH); FELBER, Armin, 6003 Luzern (CH); RIGERT, Mario, 6033 Buchrain (CH)
(74) Vertreter: Grünecker Patent- und Rechtsanwälte PartG mbB
(86) Internationale Anmeldenummer: PCT/EP2019/071744
(87) Internationale Veröffentlichungsnummer: WO 2020/035504

(56) Entgegenhaltungen:
- EP-A1- 3 533 479
- EP-A1- 3 536 359
- WO-A1-2014/161099
- WO-A1-2018/054758
- US-A1- 2005 228 342
- US-A1- 2016 296 681

## Beschreibung

Die vorliegende Erfindung betrifft eine Vorrichtung mit den oberbegrifflichen Merkmalen von Anspruch 1. Eine solche Vorrichtung ist aus der US 2015/0283311 A1 bekannt. Ähnliche Vorrichtungen sind aus US 2016/296681 A1, WO 2018/054758 A1, US 2005/0228342 A1, WO 2014/161099 A1, EP 3 536 359 A1 und EP 3 533 479 A1 bekannt, wobei EP 3 536 359 A1 und EP 3 533 479 A1 gemäß Art. 54(3) EPÜ als Stand der Technik gelten.

Aus der US 2015/0283311 A1 ist eine Vorrichtung umfassend eine Babyflasche und einen Flaschenaufsatz bekannt. Der Flaschenaufsatz ist mit dem Flaschenhals der Babyflasche verschraubt. In einer Ausführungsform detektiert eine Sensorik durch den Flaschenhals der Babyflasche fallende Milchtropfen. In einer anderen Ausführungsform wird die Offenstellung eines im Flaschenhals angeordneten Klappventils gemessen. So kann über die Bestimmung der Anzahl der Milchtropfen, die in die Babyflasche gelangen, bzw. der Offenstellung des Ventils ein Rückschluss auf die Inhaltsmenge der Babyflasche getroffen werden.

Dieser Rückschluss auf die Milchmenge ist mitunter ungenau, da die Größe und Form der Milchtropfen variieren kann. Eine Aufgabe der vorliegenden Erfindung ist es, die vorbekannte Vorrichtung zu verbessern. Insbesondere möchte die vorliegende Erfindung eine Vorrichtung angeben, die eine genaue und kontinuierliche Erfassung eines Parameters der in die Babyflasche einfließenden Milch ermöglicht, sich leicht reinigen lässt und eine einfache Wartung gewährleistet.

Zur Lösung dieser Aufgabenstellung schlägt die vorliegende Erfindung eine Vorrichtung mit den Merkmalen von Anspruch 1 vor. Die Vorrichtung umfasst eine Babyflasche und einen an die Babyflasche angekoppelten Flaschenaufsatz. Der Flaschenaufsatz weist einen Konnektor auf, an den eine Brustpumpe angeschlossen werden kann. Die Brustpumpe umfasst in der Regel eine an die Form einer menschlichen weiblichen Brust angepasste und insbesondere dichtend daran anlegbare Brusthaube, die daran angepasst ist, mit einer Handpumpe oder einer elektrisch betriebenen Pumpe zusammenzuwirken, um einen Unterdruck zwischen der Brusthaube und der Brust zu erzeugen. Der Unterdruck wird üblicherweise in einer bestimmten Frequenz entsprechend den Pump- bzw. Saughüben der Pumpe erzeugt. Dadurch wird der Milchfluss stimuliert. Der Konnektor weist meist einen Kanal auf, der in der Brusthaube aufgefangene Milch in Richtung eines Messkopfes des Flaschenaufsatzes abführt. Die Brusthaube kann auch fest in den Konnektor integriert sein.

Der Flaschenaufsatz kann von der Babyflasche entkoppelt werden, sodass die gefüllte Babyflasche mit einem Trinksauger zur Fütterung eines Babys verbunden werden kann. Der Flaschenaufsatz ist lösbar mit dem Flaschenhals der Babyflasche verbunden, in der Regel verschraubt. Bei der erfindungsgemäßen Vorrichtung weist der Flaschenaufsatz einen Messkopf mit einem Reservoir für Muttermilch auf, wobei der Messkopf eine dem Volumen des Reservoirs zugeordnete optische Sensorik aufweist. Die Sensorik muss dabei nicht das gesamte Volumen des Reservoirs erfassen.

Der Flaschenaufsatz ist an einer Öffnung der Babyflasche vorgesehen, welche für gewöhnlich von einem Flaschenhals der Babyflache ausgebildet wird. In der Regel ist auf dem dem Flaschenhals gegenüberliegenden Ende der Flaschenboden angeordnet. Für gewöhnlich ist eine Babyflasche derart ausgebildet, dass sie auf dem Flaschenboden frei und aufrecht stehen kann. Richtungsangaben wie oben, unten, vertikal, horizontal etc. beziehen sich jedenfalls auf eine mit dem Flaschenboden auf einer ebenen, horizontalen Oberfläche stehenden Vorrichtung.

Die erfindungsgemäße Babyflasche ist in der Regel aus einem zumindest teilweise transparenten thermoplastischen Kunststoff oder aus Glas hergestellt. Besonders bevorzugt wird Polypropylen verwendet. Die Babyflasche aus Kunststoff kann als Einwegflasche oder als Mehrwegflasche produziert sein und hat meist ein Gewicht von 7,5 bis 30 g. Die Mehrweg-Babyflasche hat vorzugsweise eine Wandstärke von ca. 0,9 mm.

Das Nennvolumen der Babyflasche beträgt üblicherweise 80 bis 250 ml, insbesondere 80 ml, 150 ml oder 250 ml. Das Maximalvolumen, das die Babyflasche aufnehmen kann, ist in der Regel nicht größer als 330 ml.

Die Babyflasche weist bevorzugt meist eine Höhe von ca. 60 bis 160 mm auf, vorzugsweise 66 mm, 99,5 mm, 102 mm, 136 mm oder 148,5 mm. Der Durchmesser des Flaschenhalses ist in der Regel 33 mm. Üblicherweise ist der maximale Durchmesser der Babyflasche nicht größer als 50 bis 70 mm, bevorzugt nicht größer als 53 mm, 60 mm oder 65 mm. Sämtliche Maße sind mit einer Toleranz von ± 10%, bevorzugt ± 5% zu verstehen.

In der Regel hat die Babyflasche einen im Wesentlichen zylindrischen Flaschenbauch, der sich zu einem Flaschenhals konisch verjüngt. Der Durchmesser des Flaschenbauchs kann aber auch über seine Länge hinweg variieren. Beispielsweise kann der Flaschenbauch mehrere zylindrische Abschnitte unterschiedlichen Durchmessers aufweisen, die insbesondere über einen oder mehrere konisch geformte Abschnitte verbunden sein können. Der Flaschenhals hat üblicherweise den geringsten Durchmesser. Vorzugsweise ist der Flaschenhals mit einem Außengewinde versehen.

Der Flaschenboden bildet für gewöhnlich eine Standfläche aus, auf der die Babyflasche auf einer ebenen Auflagefläche aufrecht steht. Die Standfläche ist für gewöhnlich eine im Wesentlichen ebene Fläche, die ggf. eine zentrale Wölbung nach innen in Richtung des Reservoirs aufweisen kann. Die Standfläche kann auch durch einen nach unten vorstehenden Ring gebildet sein.

Der Messkopf ist üblicherweise unterhalb des Konnektors angeordnet und fängt die vorzugsweise über eine Brustpumpe in den Konnektor gesogenen Milchtropfen in dem Reservoir auf. Dazu ist das Reservoir für gewöhnlich mit einer Einfüllöffnung am oberen Ende ausgebildet. Bevorzugt ist das Reservoir nach oben hin offen. Des Weiteren ist das Reservoir in der Regel derart ausgebildet, dass Milchtropfen nicht direkt durch das Reservoir hindurch fallen können. Vorzugsweise ist die untere Seitenwand des Reservoirs fluiddicht. Die Geometrie des Reservoirs definiert einen Hohlraum, der ein bestimmtes Volumen hat. Dieses Volumen ist in der Regel zwischen 0,5 ml und 4 ml bevorzugt 0,8 ml bis 1,5 ml. Diesem Volumen ist die Sensorik zugeordnet. Mit anderen Worten ist die Sensorik auf den Hohlraum des Reservoirs ausgerichtet. Die Sensorik ist insbesondere dazu ausgelegt, einen Parameter der Milch in dem Reservoir zu bestimmen, beispielsweise die Milchfüllmenge im Reservoir.

Die vorliegende Erfindung ermöglicht die Bestimmung eines Parameters der Milch bevor sie in die Babyflasche gelangt. Die Genauigkeit ist im Vergleich zum Stand der Technik erhöht, da die Milch nicht während des Fallens und/oder Tropfen für Tropfen gemessen werden muss. Vielmehr kann eine Vielzahl an Milchtropfen zu einer Portion im Reservoir zusammengeführt werden, die dann von der Sensorik ge- bzw. vermessen wird.

Das Reservoir weist eine Ausflussöffnung zur Babyflasche und ein in der Ausflussöffnung angeordnetes Verschlusselement auf. Dabei ist das Verschlusselement derart eingerichtet, dass es mindestens eine bestimmte Milchmenge im Reservoir zurückhält. Vorzugsweise ist das Verschlusselement als ein Ventil ausgebildet, das während einer tröpfchenweise Befüllung in einer geschlossenen Stellung an der Ausflussöffnung des Reservoirs anliegt. Übersteigt der Fluiddruck im Reservoir einen Widerstandsgrenzwert des Verschlusselements, wird das Verschlusselement in eine Offenstellung gedrückt, sodass eine gewisse Menge Milch aus dem Reservoir in die Babyflasche abfließen kann. Das Verschlusselement kann beispielsweise gemäß der auf die Anmelderin zurückgehenden WO 2014/161099 A1 oder nach Art eines Klappventils gemäß US 2015/0283311 A1 ausgebildet sein. Üblicherweise entleert sich das Reservoir nicht komplett, da eine Abnahme des Fluiddrucks bewirkt, dass das Verschlusselement sich wieder in die Geschlossenstellung versetzt.

Der Widerstandsgrenzwert des Verschlusselements ist vorzugsweise einstellbar. Gegebenenfalls ist das Verschlusselement gegen die Ausflussöffnung vorgespannt. Bevorzugt liegt die Füllhöhe zwischen 15 und 25 mm. Die Restmenge, die üblicherweise nach einem Wiederverschließen des Verschlusselements im Reservoir verbleibt, beträgt für gewöhnlich 0,1 ml bis 0,3 ml.

In der Regel ist der Fluiddruck der Milch im Reservoir auf das Verschlusselement ein hydrostatischer Druck. Das Ventil ist vorzugsweise als Ein-Weg-Ventil ausgebildet. Weiter bevorzugt weist das Ventil eine Ventilmembran auf, die aufgrund ihrer Geometrie bei einem Fluiddruck von Seiten der Babyflasche auf die Ventilmembran in der Ruhestellung verbleibt. In der Ruhestellung liegt die Ventilmembran an der Ausflussöffnung des Reservoirs an. Für gewöhnlich ist die Ausflussöffnung an einer Seitenwand des Reservoirs vorgesehen, sodass die Ventilmembran von der Vertikalen weg in die Offenstellung gedrückt, vorzugsweise verschwenkt wird. Die Ventilmembran ist vorzugsweise aus einem flexiblen Kunststoff, insbesondere Silikon, gefertigt.

So kann auf einfache und energiesparende Weise ein Fließrhythmus, hervorgerufen durch die Vakuumzyklen der Pumpe, d.h. ein portionsweises Abfließen der Milch in die Babyflasche implementiert werden.

Dem Reservoir sind zumindest zwei sich höhengleich gegenüberliegend angeordnete optische Sensormittel zugeordnet. Die Bezeichnungen "optisch" sowie "Licht" sind in diesem Zusammenhang nicht auf das sichtbare Licht beschränkt, sondern beziehen sich grundsätzlich auf das gesamte elektromagnetische Spektrum. Vorzugsweise sind die optischen Sensormittel auf den Infrarotbereich angepasst. In der Regel sind die Sensormittel so angeordnet, dass wenn Milch das Volumen des Reservoirs ausfüllt, zumindest ein Teil der Milchsäule im Reservoir zwischen den Sensormitteln vorgesehen ist. Üblicherweise sind die Sensormittel derart eingerichtet, dass sie ein unterschiedliches Signal erfassen, je nachdem ob zwischen den Sensormitteln Milch oder Luft angeordnet ist. Vorzugsweise sind die Sensormittel in sich gegenüberliegende Seitenwände des Reservoirs, beispielsweise mittels Spritzgießen, integriert. Alternativ können die Sensormittel jeweils außen an einer transparenten bzw. transluzenten Seitenwand am Reservoir angebracht sein.

So kann festgestellt werden, ob sich eine Milchsäule im Reservoir bis zwischen die Sensormittel erstreckt bzw. wie hoch sich diese erstreckt, wodurch ein Rückschluss auf die Milchfüllmenge im Reservoir getroffen werden kann.

Lichtdetektoren auf einer Seite des Reservoirs und Lichtemitterer auf einer anderen Seite des Reservoirs sind jeweils in Höhenrichtung übereinander angeordnet. Je ein Lichtdetektor und je ein Lichtemitter sind dabei höhengleich angeordnet, sodass sich der kürzeste Lichtpfad zwischen Lichtdetektor und Lichtemitter in horizontaler Richtung erstreckt. Der Lichtemitter ist in der Regel auf den Lichtdetektor ausgerichtet. Das von dem Lichtdetektor empfangene Signal hat eine höhere Intensität, wenn zwischen Lichtemitter und Lichtdetektor Luft vorhanden ist, als wenn zwischen Lichtemitter und Lichtdetektor Milch vorhanden ist.

Der Lichtemitter ist vorzugsweise eine LED oder ein LED-Chip und der Lichtdetektor vorzugsweise ein Phototransistor. Vorzugsweise sind die Lichtemitter und die Lichtdetektoren jeweils in einer geraden, sich in Höhenrichtung erstreckenden Zeile angeordnet.

So kann der Pegel der Milchsäule im Reservoir genauer bestimmt werden.

Nach einer bevorzugten Weiterbildung der vorliegenden Erfindung weist die Vorrichtung eine steuerungsmäßig mit den Lichtemittern verbundene Steuereinheit auf, die derart eingerichtet ist, dass die Lichtemitter in einer vorbestimmten Zeitspanne und in einer vorbestimmten Reihenfolge nacheinander ein- und wieder ausgeschaltet werden können. Die Zeitspanne beträgt in der Regel wenige Millisekunden, bevorzugt nicht mehr als 2 Millisekunden. Da die Zeitspanne sehr kurz gewählt ist, kann durch das Auftragen der gemessenen Intensität der Lichtdetektoren gegen die Höhe der jeweiligen Lichtdetektoren ein Abbild der Milchsäule im Reservoir zu dem jeweiligen Zeitpunkt erstellt werden. Die Reihenfolge, nach der die Lichtemitter ein- und wieder ausgeschaltet werden, ist üblicherweise weniger wichtig, vorzugsweise jedoch in Höhenrichtung sequenziell.

Vorzugsweise erzeugt die Steuereinheit ein solches Abbild in periodischen Zeitabständen. Nach dieser bevorzugten Weiterbildung ist die Steuereinheit derart eingerichtet, dass sich das Ein- und Ausschalten der Lichtemitter periodisch wiederholt. Die Periode beträgt vorzugsweise zwischen 10 und 20 Millisekunden, weiter bevorzugt zwischen 13 und 17 Millisekunden, sehr bevorzugt ca. 15 Millisekunden.

So kann der Pegel der Milchsäule im Reservoir kontinuierlich verfolgt werden.

Nach einer weiteren bevorzugten Weiterbildung der vorliegenden Erfindung weist die Vorrichtung eine Recheneinheit auf, die dazu eingerichtet ist, mittels einer gemessenen Intensität der Lichtdetektoren einen Parameter der in dem Reservoir enthaltenen Milch zu bestimmen. Beispielsweise lässt sich mit der Recheneinheit anhand der Änderung des Milchpegels im Reservoir in Abhängigkeit der Zeit der Volumenstrom in die Babyflasche berechnen. Dies lässt einen direkten Rückschluss auf die Milchabgabe der Brust im zeitlichen Verlauf zu. Ein anderer Parameter ist die Füllmenge an Milch im Reservoir zu einem bestimmten Zeitpunkt, die über den aktuellen Pegel der Milchsäule und die Maße des Reservoirs bestimmt werden kann.

Vorzugsweise stimmt eine zentrale Achse des Reservoirs mit einer zentralen Achse des Messkopfes überein. Damit kann die Sensorik optimal auf das Reservoir ausgerichtet werden. Des Weiteren kann zwischen dem Reservoir und einer Außenwand des Messkopfes die Steuereinheit und die Recheneinheit angeordnet werden.

Weiter bevorzugt weist das Reservoir einen im Wesentlichen rechteckigen, insbesondere quadratischen Querschnitt auf. Der Querschnitt verläuft hierbei in der Horizontalen, sodass sich die rechteckige Form bei einer Draufsicht auf den Querschnitt bzw. das Reservoir zeigt. Dies vereinfacht die Anlage des Verschlusselements an der Ausflussöffnung, sofern die Ausflussöffnung an einer Seitenwand des Reservoirs und das Verschlusselement als ein ebenes, plättchenartiges Element vorgesehen ist. Bevorzugt hat das Reservoir eine Länge von 8 mm, eine Breite von 8 mm und eine Höhe von 20 mm. Die Auslassöffnung hat vorzugsweise einen Durchmesser von 5 mm.

Nach einer bevorzugten Weiterbildung der vorliegenden Erfindung umfasst der Messkopf ein äußeres Gehäuse und ein das Reservoir ausbildendes inneres Gehäuse, wobei das äußere Gehäuse lösbar mit dem inneren Gehäuse verbunden ist. In der Regel ist das innere Gehäuse koaxial in dem äußeren Gehäuse angeordnet.

Dadurch vereinfachen sich die Reinigung des Messkopfes und die Wartung der Sensorik. Das äußere Gehäuse erhöht den Schutz des Reservoirs gegen Stöße und der Sensorik vor Beschädigungen.

Vorzugsweise ist das äußere Gehäuse über ein Gewinde mit dem Hals der Babyflache verschraubt. In der Regel weist der Flaschenhals der Babyflasche ein Außengewinde auf, das mit einem Innengewinde des äußeren Gehäuses zusammenwirkt. Weiter bevorzugt ist das innere Gehäuse über ein Gewinde mit dem Konnektor veschraubbar. Üblicherweise weist das innere Gehäuse ein Außengewinde auf, das von dem Konnektor umfänglich umgeben ist und mit diesem im Gewindeeingriff steht.

Dadurch können die einzelnen Bestandteile des Flaschenaufsatzes leicht voneinander getrennt werden, sodass sich die Reinigung und die Wartung vereinfacht.

Der Messkopf kann alternativ einteilig ausgebildet und auf der einen Seite mit dem Hals der Babyflasche und auf der anderen Seite mit dem Konnektor lösbar verbunden, vorzugsweise verschraubt sein. Gemäß einer anderen Alternative kann der Flaschenaufsatz insgesamt einteilig ausgebildet sein.

Das Reservoir besteht bevorzugt aus Polypropylen (PP), wobei das Reservoir insbesondere für infrarotes Licht durchlässig sein sollte. Das innere und das äußere Gehäuse sollten ebenfalls aus Polypropylen (PP) bestehen.

In einem nebengeordneten Aspekt gibt die vorliegende Erfindung einen Flaschenaufsatz für eine Babyflasche an. Dieser hat einen Konnektor für eine Brustpumpe und einen Messkopf mit einem Reservoir für Muttermilch, das eine Ausflussöffnung zur Babyflasche und ein in der Ausflussöffnung angeordnetes Verschlusselement aufweist, wobei das Verschlusselement derart eingerichtet ist, dass es mindestens eine bestimmte Milchmenge im Reservoir zurückhält. Der Messkopf weist eine dem Volumen des Reservoirs zugeordnete optische Sensorik auf. Dem Reservoir sind zumindest zwei sich höhengleich gegenüberliegend angeordnete optische Sensormittel zugeordnet, wobei Lichtdetektoren auf einer Seite des Reservoirs und Lichtemitter auf einer anderen Seite des Reservoirs jeweils in Höhenrichtung übereinander angeordnet sind. Vorzugsweise ist der Flaschenaufsatz nach einer oder mehreren der vorstehend diskutierten Weiterbildungen ausgebildet.

Weitere Einzelheiten der vorliegenden Erfindung ergeben sich aus der nachfolgenden Beschreibung eines Ausführungsbeispiels der Erfindung in Verbindung mit der Zeichnung. In dieser zeigen:
- Figur 1: eine Seitenansicht eines Ausführungsbeispiels nach der vorliegenden Erfindung,
- Figur 2: eine Schnittansicht des Messkopfes aus Figur 1,
- Figur 3: eine Schnittansicht des inneren Gehäuses des Messkopfes aus Figur 2 aus einer im Vergleich zu Figur 2 um 90° gedrehten Perspektive,
- Figur 4: eine Schnittansicht des inneren Gehäuses des Messkopfes aus einer im Vergleich zu Figur 3 um 180° gedrehten Perspektive, und
- Figur 5: eine Teilschnittansicht des Ausführungsbeispiels.

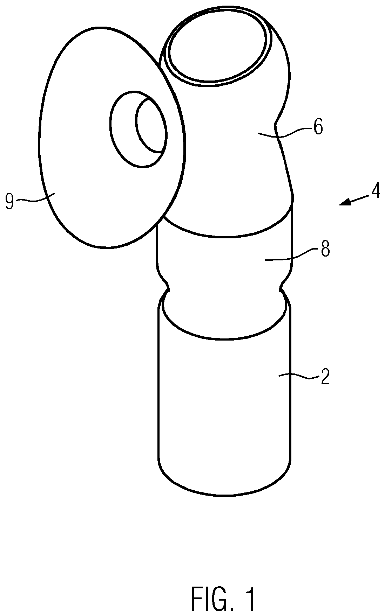

Die Figur 1 zeigt ein Ausführungsbeispiel der erfindungsgemäßen Vorrichtung, umfassend eine Babyflasche 2 und einen an die Babyflache 2 angekoppelten Flaschenaufsatz 4. Der Flaschenaufsatz 4 weist einen Konnektor 6 und einen zwischen der Babyflasche 2 und dem Konnektor 6 angeordneten Messkopf 8 auf. Der Messkopf 8 ist mit der Babyflasche 2 und dem Konnektor 6 verschraubt. Der Konnektor 6 weist üblicherweise einen Anschluss für eine Brustpumpe auf. Von dieser Brustpumpe ist in der Figur 1 lediglich eine Brusthaube 9 dargestellt, die an eine weibliche Brust anlegbar ist und im Zusammenwirken mit einer an dem Konnektor 6 anschließbaren Pumpe einen Unterdruck an der Brust erzeugen kann. Die Babyflasche 2 ist nach diesem Ausführungsbeispiel rotationssymmetrisch um eine vertikale Symmetrieachse, die mit einer zentralen Achse des Messkopfes übereinstimmt. Die Babyflasche 2 ist aus einem transparenten Kunststoff oder Glas hergestellt.

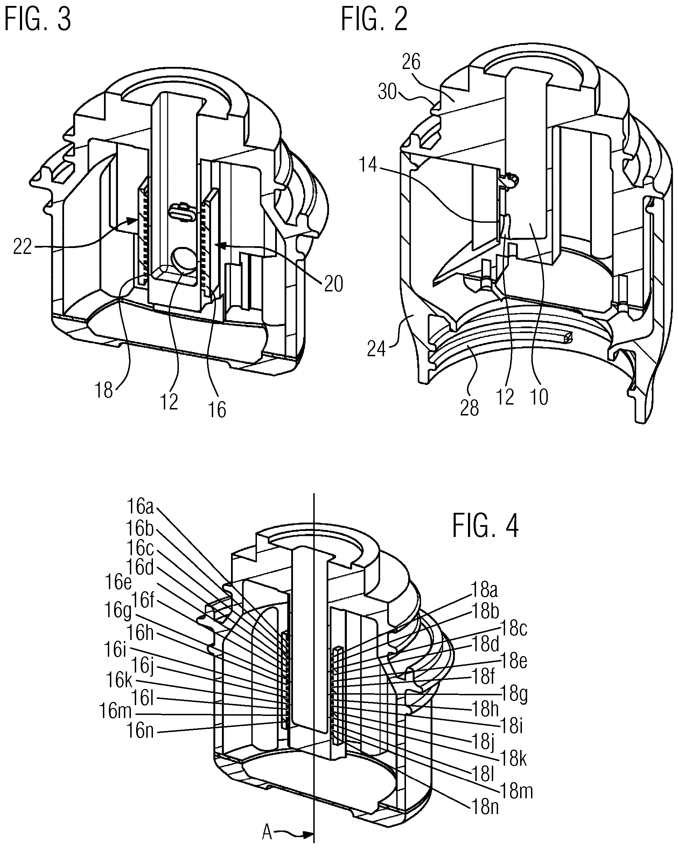

Die Figur 2 zeigt eine Schnittansicht des Messkopfes 8, um dessen zentrale Achse A (s. Fig. 4) ein Reservoir 10 angeordnet ist. Das Reservoir 10 hat nach dem Ausführungsbeispiel einen quadratischen Querschnitt. Am unteren Ende der in der Figur linken Seitenwand des Reservoirs 10 weist dieses eine Ausflussöffnung 12 auf. Der untere Boden des Reservoirs 10 ist in Richtung der Auslassöffnung 12 absinkend ausgebildet. An der Auslassöffnung liegt ein Verschlusselement 14 an. Das Verschlusselement 14 ist in dem Ausführungsbeispiel als Ventilmembran ausgebildet. Bei ausreichendem Fluiddruck von Milch im Reservoir auf die Ventilmembran kann die Ventilmembran seitlich von der Auslassöffnung 12 abheben und diese zumindest teilweise freigeben. Die Auslassöffnung 12 ist bündig mit dem untersten Punkt des Reservoirs 10 ausgebildet.

Wie die Figur 3 zeigt, sind dem Reservoir höhengleich gegenüberliegend angeordnete Lichtdetektoren 16 und Lichtemitter 18 zugeordnet. Die Lichtdetektoren 16 sind dabei auf einer Seite 20 des Reservoirs in einer vertikalen Zeile in Höhenrichtung übereinander angeordnet. Die Lichtemitter 18 sind entsprechend auf der gegenüberliegenden Seite 22 des Reservoirs 10 in einer vertikalen Zeile in Höhenrichtung übereinander angeordnet. Die Auslassöffnung ist in der Figur 3 als im Wesentlichen kreisrunde Öffnung zu erkennen.

Der Messkopf 8 weist ein äußeres Gehäuse 24 und ein das Reservoir 10 ausbildendes inneres Gehäuse 26 auf (s. Figur 2). Über ein Innengewinde 28 am unteren Ende des äußeren Gehäuses 24 ist der Messkopf 8 mit einem Außengewinde am Flaschenhals der Babyflasche 2 verschraubt. Ein Außengewinde 30 ist am inneren Gehäuse 26 des Messkopfes 8 ausgebildet, über das der Messkopf 8 mit dem Konnektor 6 verschraubt ist.

Figur 4 verdeutlicht die höhengleiche Anordnung der Lichtdetektoren 16 und der Lichtemitter 18 beidseits der vertikalen Symmetrieachse A des Messkopfes 8. Nach diesem Ausführungsbeispiel sind insgesamt 14 Lichtdetektoren 16a bis 16n und 14 Lichtemitter 18a bis 18n vorgesehen. Diese sind jeweils linear übereinander in einer vertikalen Zeile parallel zu der zentralen Achse A angeordnet. Die Lichtdetektoren 16 und die Lichtemitter 18 sind jeweils in einer Seitenwand des Reservoirs 10 befestigt. Das Reservoir 10 ist aus einem transparenten Material hergestellt.

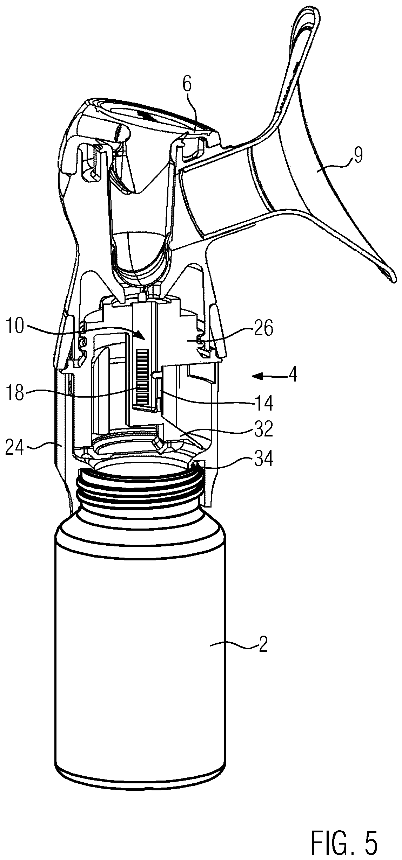

Wie Figur 5 zeigt, schließt sich an die Auslassöffnung 12 des Reservoirs 10 eine in Richtung auf das äußere Gehäuse 24 abschüssige Rampe 32 an. Das äußere Gehäuse 24 weist einen die Öffnung am Flaschenhals umgreifenden Kragen 34 auf, der als Abtropfkante dient.

Muttermilch, welche in der Brusthaube 9 aufgefangen wird, wird über einen mit der Brusthaube einteilig geformten Stutzen in den Konnektor 6 geleitet. Der Stutzen verläuft von der Brusthaube 9 schräg nach unten hin zum Konnektor 6. Ein Kanal im Konnektor 6 ist unterhalb des unteren Endes des Stutzens ausgebildet. Der Kanal endet direkt über dem nach oben hin offenen Reservoir. So fließt in der Brusthaube 9 aufgefangene Milch aufgrund der soeben beschriebenen Geometrie und der Schwerkraft in das Reservoir 10. Der abschüssige Boden des Reservoirs (s. Fig. 2) bewirkt, dass sich die Milch zuerst vor der Ausflussöffnung 12 sammelt. Das Verschlusselement 14 hält dem Fluiddruck der Milch, der aus einer Gewichtskraft resultiert, zunächst stand. Es bildet sich eine Milchsäule im Reservoir 10. Ist der Fluiddruck der Milchsäule groß genug, um das Verschlusselement 14 von der Ausflussöffnung 12 zu drücken, kann eine Portion der Milchsäule durch die Ausflussöffnung 12 aus dem Reservoir 10 abfließen. Dabei fließt die Portion über die Rampe 32 auf eine Innenwand des äußeren Gehäuses. Von dieser Innenwand fließt die Portion nach unten zu dem Kragen 34, an welchem die Portion schließlich in die Babyflasche 2 abtropft. Versetzt sich das Verschlusselement danach in die Geschlossenstellung, steigt die Milchsäule im Reservoir wieder, bis das Verschlusselement erneut in die Offenstellung gedrückt wird.

Nach dem Ausführungsbeispiel wird die Milchsäule im Reservoir 10 alle 15ms von den Sensormitteln 16, 18 durchleuchtet. Die Sensormittel 16, 18 benötigen für dieses Durchleuchten nicht mehr als 2ms. So entsteht alle 15ms eine Momentaufnahme der Höhenerstreckung der Milchsäule. Dabei messen die Lichtdetektoren 16, die oberhalb der Milchsäule angeordnet sind eine höhere Intensität, als die Lichtdetektoren 16, zwischen denen sich die Milchsäule erstreckt, da Milch mehr Licht absorbiert und/oder streut als Luft. Die Lichtdetektoren 16 und Lichtemitter 18 nach dem Ausführungsbeispiel sind indes auf den Infrarot-Bereich angepasst.

Die Höhe der Milchsäule im Reservoir 10 wird so kontinuierlich überwacht. Bei jeder Offenstellung des Verschlusselements 14 nimmt die Höhe der Milchsäule bzw. der Milchpegel ab. Aus dieser Abnahme kann die Milchmenge bzw. die Portion berechnet werden, die über einen Vakuumzyklus der saugenden Pumpe in die Babyflasche abfließt. Da die Überwachung kontinuierlich ist, kann so der Volumenstrom in die Babyflasche, d.h. die Milchmenge pro Zeit, ermittelt werden. Ist der Volumenstrom hoch, wird mehr Milch abgepumpt. Ist der Volumenstrom niedrig, wird weniger Milch abgepumpt.

### Bezugszeichenliste

- 2: Babyflasche
- 4: Flaschenaufsatz
- 6: Konnektor
- 8: Messkopf
- 10: Reservoir
- 12: Ausflussöffnung
- 14: Verschlusselement
- 16: Lichtdetekor
- 18: Lichtemitter
- 20, 22: gegenüberliegende Seiten des Reservoirs
- 24: äußeres Gehäuse
- 26: inneres Gehäuse
- 28: Innengewinde
- 30: Außengewinde
- 32: abschüssige Rampe
- 34: Abtropfkante
- A: zentrale Achse

## Patentansprüche

1. Vorrichtung, umfassend eine Babyflasche (2) und einen an die Babyflasche (2) angekoppelten Flaschenaufsatz (4), der einen Konnektor (6) für eine Brustpumpe und einen Messkopf (8) mit einem Reservoir (10) für Muttermilch aufweist, wobei das Reservoir (10) eine Ausflussöffnung (12) zur Babyflasche (2) und ein in der Ausflussöffnung (12) angeordnetes Verschlusselement (14) aufweist, und wobei das Verschlusselement (14) derart eingerichtet ist, dass es mindestens eine bestimmte Milchmenge im Reservoir (10) zurückhält, **dadurch gekennzeichnet, dass** der Messkopf (8) eine dem Volumen des Reservoirs (10) zugeordnete optische Sensorik aufweist, wobei dem Reservoir (10) zumindest zwei sich höhengleich gegenüberliegend angeordnete optische Sensormittel (16, 18) zugeordnet sind und wobei Lichtdetektoren (16) auf einer Seite (20) des Reservoirs (10) und Lichtemitter (18) auf einer anderen Seite (22) des Reservoirs (10) jeweils in Höhenrichtung übereinander angeordnet sind.

2. Vorrichtung nach Anspruch 1, **dadurch gekennzeichnet, dass** das Verschlusselement (14) ein Ventil (14) ist, das in einer Geschlossenstellung an der Ausflussöffnung (12) des Reservoirs (10) anliegt und mittels einer Gewichtskraft von in dem Reservoir (10) enthaltener Milch in eine Offenstellung gedrängt wird.

3. Vorrichtung nach Anspruch 1 oder 2, **gekennzeichnet durch** eine steuerungsmäßig mit den Lichtemittern (18) verbundene Steuereinheit, die derart eingerichtet ist, dass die Lichtemitter (18) in einer vorbestimmten Zeitspanne und in einer vorbestimmten Reihenfolge nacheinander ein- und wieder ausgeschaltet werden.

4. Vorrichtung nach Anspruch 3, **dadurch gekennzeichnet, dass** die Steuereinheit derart eingerichtet ist, dass sich das Ein- und Ausschalten der Lichtemitter (18) periodisch wiederholt.

5. Vorrichtung nach einem der vorherigen Ansprüche, **gekennzeichnet durch** eine Recheneinheit, die dazu eingerichtet ist, mittels einer gemessenen Intensität der Lichtdetektoren (16) einen Parameter der in dem Reservoir (10) enthaltenen Milch zu bestimmen.

6. Vorrichtung nach einem der vorherigen Ansprüche, **dadurch gekennzeichnet, dass** eine zentrale Achse (A) des Reservoirs mit einer zentralen Achse (A) des Messkopfes übereinstimmt.

7. Vorrichtung nach einem der vorherigen Ansprüche, **dadurch gekennzeichnet, dass** das Reservoir (10) einen im Wesentlichen rechteckigen Querschnitt aufweist.

8. Vorrichtung nach einem der vorherigen Ansprüche, **dadurch gekennzeichnet, dass** das Reservoir (10) einen im Wesentlichen quadratischen Querschnitt aufweist.

9. Vorrichtung nach einem der vorherigen Ansprüche, **dadurch gekennzeichnet, dass** der Messkopf (8) ein äußeres Gehäuse (24) und ein das Reservoir (10) ausbildendes inneres Gehäuse (26) umfasst und dass das äußere Gehäuse (24) lösbar mit dem inneren Gehäuse (26) verbunden ist.

10. Vorrichtung nach Anspruch 9, **dadurch gekennzeichnet, dass** das äußere Gehäuse (24) über ein Gewinde (28) mit dem Hals der Babyflasche (2) verschraubbar ist.

11. Vorrichtung nach Anspruch 9 oder 10, **dadurch gekennzeichnet, dass** das innere Gehäuse (26) über ein Gewinde (30) mit dem Konnektor verschraubbar ist.

12. Flaschenaufsatz (4) für eine Babyflasche (2), der einen Konnektor (6) für eine Brustpumpe und einen Messkopf (8) mit einem Reservoir (10) für Muttermilch aufweist, das eine Ausflussöffnung (12) zur Babyflasche (2) und ein in der Ausflussöffnung (12) angeordnetes Verschlusselement (14) aufweist, wobei das Verschlusselement (14) derart eingerichtet ist, dass es mindestens eine bestimmte Milchmenge im Reservoir (10) zurückhält, **dadurch gekennzeichnet, dass** der Messkopf (8) eine dem Volumen des Reservoirs (10) zugeordnete optische Sensorik aufweist, wobei dem Reservoir (10) zumindest zwei sich höhengleich gegenüberliegend angeordnete optische Sensormittel (16, 18) zugeordnet sind und wobei Lichtdetektoren (16) auf einer Seite (20) des Reservoirs (10) und Lichtemitter (18) auf einer anderen Seite (22) des Reservoirs (10) jeweils in Höhenrichtung übereinander angeordnet sind.

## Claims

1. An apparatus comprising a baby bottle (2) and a bottle top attachment (4) coupled to said baby bottle (2) and having a connector (6) for a breast pump, wherein said bottle top attachment (4) comprises a measuring head (8) with a reservoir (10) for breast milk, said reservoir (10) comprising an outflow opening (12) to said baby bottle (2) and a closure element (14) disposed in said outflow opening (12), and said closure element (14) being adapted such that it retains at least a certain amount of milk in said reservoir (10), **characterized in that** said measuring head (8) comprises an optical sensor unit associated with a volume of said reservoir (10), wherein light detectors (16) are arranged one above another in a height direction on one side of said reservoir (10) and light emitters (18) are arranged one above another in the height direction on another side of said reservoir (10), and wherein each light detector (16) on the one side of the reservoir (10) is assigned to one light emitter (18) arranged on the other side of the reservoir (10) at the same height.

2. The apparatus according to claim 1, **characterized in that** the closure element (14) is a valve (14) that is arranged in said outflow opening (12), and in a closed position bears against said outflow opening (12) of said reservoir (10) and is forced to an open position due to a weight force of milk contained in said reservoir (10).

3. The apparatus according to claim 1 or 2, **characterized by** a control unit that is connected in a controlling manner to said light emitters (18) and adapted such that said light emitters (18) are switched on and off during a predetermined period of time and in a predetermined sequence.

4. The apparatus according to claim 3, **characterized in that** said control unit is adapted such that switching said light emitters (18) on and off repeats periodically.

5. The apparatus according to one of the preceding claims, **characterized by** a processing unit which is adapted to determine a parameter of the milk contained in said reservoir (10) by way of intensity measured by said light detectors (16).

6. The apparatus according to one of the preceding claims, **characterized in that** a central axis (A) of said reservoir coincides with a central axis (A) of said measuring head.

7. The apparatus according to one of the preceding claims, **characterized in that** said reservoir (10) has a substantially rectangular cross-section.

8. The apparatus according to one of the preceding claims, **characterized in that** said reservoir (10) has a substantially square cross-section.

9. The apparatus according to one of the preceding claims, **characterized in that** said measuring head (8) comprises an outer housing (24) and an inner housing (26) forming said reservoir (10) and said outer housing (24) is releasably connected to said inner housing (26).

10. The apparatus according to claim 9, **characterized in that** said outer housing (24) is screwed to a neck of said baby bottle (2) by way of a thread (28).

11. The apparatus according to claim 9 or 10, **characterized in that** said inner housing (26) is screwed to said connector by way of a thread (30).

12. A bottle top attachment (4) for a baby bottle (2) having a connector (6) for a breast pump, wherein said bottle top attachment (4) comprises a measuring head (8) with a reservoir (10) for breast milk, said reservoir (10) comprising an outflow opening (12) to said baby bottle (2) and a closure element (14) disposed in said outflow opening (12), and said closure element (14) being adapted such that it retains at least a certain amount of milk in said reservoir (10), **characterized in that** said measuring head (8) comprising an optical sensor unit associated with a volume of said reservoir (10), wherein light detectors (16) are arranged one above another in a height direction on one side of said reservoir (10) and light emitters (18) are arranged one above another in the height direction on another side of said reservoir (10), and wherein each light detector (16) on the one side of the reservoir (10) is assigned to one light emitter (18) arranged on the other side of the reservoir (10) at the same height.

## Revendications

1. Dispositif comprenant un biberon (2) et un embout de biberon (4) couplé au biberon (2), qui présente un connecteur (6) pour un tire-lait et une tête de mesure (8) avec un réservoir (10) pour le lait maternel, le réservoir (10) présentant une ouverture d'écoulement (12) vers le biberon (2) et un élément de fermeture (14) disposé dans l'ouverture d'écoulement (12), et l'élément de fermeture (14) étant agencé de telle sorte qu'il retient au moins une certaine quantité de lait dans le réservoir (10), **caractérisé en ce que** la tête de mesure (8) présente un système de détection optique associé au volume du réservoir (10), au moins deux moyens de détection optique (16, 18) disposés en face l'un de l'autre à la même hauteur étant associés au réservoir (10), et des détecteurs de lumière (16) étant disposés sur un côté (20) du réservoir (10) et des émetteurs de lumière (18) étant disposés sur un autre côté (22) du réservoir (10), respectivement l'un au-dessus de l'autre dans le sens de la hauteur.

2. Dispositif selon la revendication 1, **caractérisé en ce que** l'élément de fermeture (14) est une soupape (14) qui, dans une position fermée, s'applique contre l'ouverture d'écoulement (12) du réservoir (10) et est poussée dans une position ouverte au moyen d'une force de poids du lait contenu dans le réservoir (10).

3. Dispositif selon la revendication 1 ou 2, **caractérisé par** une unité de commande reliée aux émetteurs de lumière (18) de la commande, qui est agencée de telle sorte que les émetteurs de lumière (18) sont successivement allumés et à nouveau éteints dans un laps de temps prédéterminé et dans un ordre prédéterminé.

4. Dispositif selon la revendication 3, **caractérisé en ce que** l'unité de commande est agencée de telle sorte que l'activation et la désactivation des émetteurs de lumière (18) se répètent périodiquement.

5. Dispositif selon l'une des revendications précédentes, **caractérisé par** une unité de calcul agencée pour déterminer, au moyen d'une intensité mesurée des détecteurs de lumière (16), un paramètre du lait contenu dans le réservoir (10).

6. Dispositif selon l'une des revendications précédentes, **caractérisé en ce qu'**un axe central (A) du réservoir coïncide avec un axe central (A) de la tête de mesure.

7. Dispositif selon l'une des revendications précédentes, **caractérisé en ce que** le réservoir (10) présente une section transversale sensiblement rectangulaire.

8. Dispositif selon l'une des revendications précédentes, **caractérisé en ce que** le réservoir (10) présente une section transversale sensiblement carrée.

9. Dispositif selon l'une des revendications précédentes, **caractérisé en ce que** la tête de mesure (8) comprend un boîtier extérieur (24) et un boîtier intérieur (26) formant le réservoir (10), et **en ce que** le boîtier extérieur (24) est relié de manière amovible au boîtier intérieur (26).

10. Dispositif selon la revendication 9, **caractérisé en ce que** le boîtier extérieur (24) peut être vissé sur le goulot du biberon (2) par l'intermédiaire d'un filetage (28).

11. Dispositif selon la revendication 9 ou 10, **caractérisé en ce que** le boîtier intérieur (26) peut être vissé sur le connecteur par l'intermédiaire d'un filetage (30).

12. Embout de biberon (4) pour un biberon (2), qui présente un connecteur (6) pour un tire-lait et une tête de mesure (8) avec un réservoir (10) pour le lait maternel, qui présente une ouverture d'écoulement (12) vers le biberon (2) et un élément de fermeture (14) disposé dans l'ouverture d'écoulement (12), l'élément de fermeture (14) étant disposé de telle sorte qu'il retient au moins une quantité déterminée de lait dans le réservoir (10), **caractérisé en ce que** la tête de mesure (8) présente un système de détection optique associé au volume du réservoir (10), au moins deux moyens de détection optique (16, 18) disposés en face l'un de l'autre à la même hauteur étant associés au réservoir (10) et des détecteurs de lumière (16) étant disposés sur un côté (20) du réservoir (10) et des émetteurs de lumière (18) étant disposés sur un autre côté (22) du réservoir (10) respectivement l'un au-dessus de l'autre dans le sens de la hauteur.
